Europäisches Patentamt

European Patent Office

Office européen des brevets

(19)

(11) Numéro de publication: **0 263 041**
**A2**

(12)

# DEMANDE DE BREVET EUROPEEN

(21) Numéro de dépôt: 87420246.8

(22) Date de dépôt: **22.09.87**

(51) Int. Cl.⁴: **A 61 F 7/10**

(30) Priorité: **22.09.86 FR 8613360**

(43) Date de publication de la demande:
**06.04.88 Bulletin 88/14**

(84) Etats contractants désignés:
**AT BE CH DE ES GB IT LI NL SE**

(71) Demandeur: **Bally, Philippe**
**2, rue de la Loge**
**F-69005 Lyon (FR)**

(72) Inventeur: **Bally, Philippe**
**2, rue de la Loge**
**F-69005 Lyon (FR)**

(74) Mandataire: **Karmin, Roger et al**
**Cabinet MONNIER 150, cours Lafayette**
**F-69003 Lyon (FR)**

(54) **Procédé raffermisseur et decongestionnant, en particulier du buste, par effet cryogénique.**

(57) Dispositif raffermisseur et décongestionnant (en particulier du buste) par effet cryogénique et utilisant un appareillage autonome analogue à un soutien-gorge dont les deux bonnets sont des orthèses mammaires muticellulaires amovibles et pré-formées (ou sont susceptibles d'en recevoir). Les cellules des orthèses mammaires sont remplies d'un fluide à haute chaleur latente de congélation. Le soutien-gorge est destiné à être porté au moins une fois par jour pendant une courte durée.

figure2

EP 0 263 041 A2

## Description

La présente invention concerne le domaine de cryothérapie et plus particulièrement celui de la tonicité et de l'élasticité de la peau du buste.

Dans les traditions ancestrales, il est reconnu que le froid et surtout l'eau "glacée" ± 6° centigrades donne de bons résultats sur la tonicité des tissus. Il n'est qu'à voir la peau tendue par "la chair de poule" lorsque nous avons froid. Tous les muscles peauciers sont contractés. Or l'on sait que les glandes mammaires n'ont, pour tout soutien que les muscles peauciers ; en les soumettant à des alternances chaud/froid, nous arrivons donc à tonifier les seins.

Il existe actuellement, des dispositifs de massages des seins avec des jets d'eau froide, rotatifs, pour éviter ou remédier à la ptose des seins, si fréquente après un accouchement, un régime amaigrissant ou tout simplement du fait de l'absence du port du soutien-gorge, la mode étant aux seins nus aussi bien sous les vêtements qu'à la plage. Il existe aussi toutes sortes de pommades permettant de redonner élasticité et tonicité. Mais si ces différentes méthodes donnent des résultats, ils sont parfois difficiles à obtenir. Souvent ces méthodes sont astreignantes, coûteuses, nécessitant soit une mise en oeuvre assez longue, soit des massages longs et fastidieux. Par ailleurs, on connait dans l'art antérieur des dispositifs autonomes de traitement des seins, du type comportant deux récipients pré-formés amovibles et pouvant contenir un liquide chaud ou froid mais dont la régulation des frigories est mal contrôlée et/ou mal répartie, ce qui occasionne pour les utilisateurs des brûlures superficielles.

La présente invention concerne un dispositif original qui a pour but de pallier ces inconvénients.

L'invention sera mieux comprise grâce à la description ci-après, qui se rapporte à un mode de réalisation préféré, donné à titre d'exemple non limitatif, et expliqué avec références aux dessins schématiques annexés, dans lesquels:

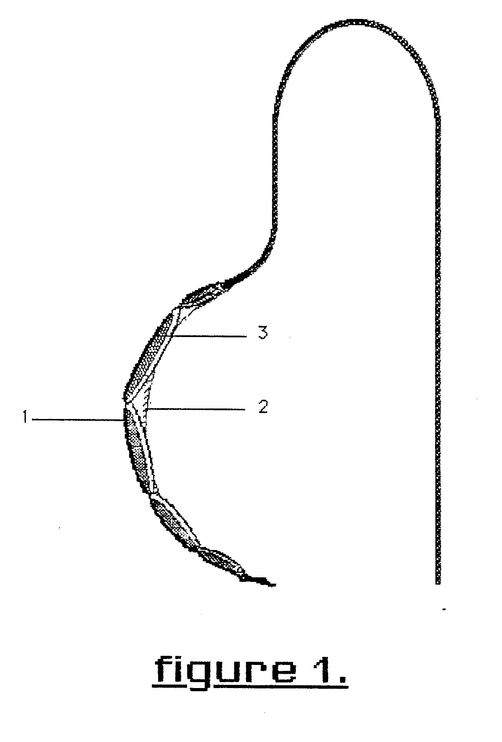

- la figure 1 est une vue en coupe simplifiée d'un appareillage conforme à l'invention;

- la figure 2 est une vue de face montrant les orthèses mammaires (3), dont 1 des bonnets, le gauche est présenté côté interne (2), et dont le droit présente un revêtement partiellement arraché. Il est a noté que l'orthèse est un appareil qui pallie une déficience corporelle d'ordre mécanique, sans constituer à la différence de la prothèse le substituant d'un élément manquant. Conformément à l'invention:

- la figure 3 est une vue de profil partiellement arrachée d'une des orthèses mammaires multicellulaires (3) conforme à l'invention.

- les figures 4 et 5 sont des représentations de différentes versions de l'invention.

Dans le procédé de traitement cryogénique des seins le dispositif employé consiste en au moins une orthèse mammaire (3) dont l'une des faces à un pouvoir isothermique supérieur à l'autre et est constitué par au moins deux feuillets (6-7) en matière plastique souple, déformable, et résistant au gel, soudés partiellement entre eux (9) de façon à créer des cellules (8) dont l'orientation assure une répartition uniforme d'un fluide (5) sur toute la surface de l'orthèse mammaire. Le dispositif est appliqué pendant une durée moyenne de 15 minutes après avoir été rechargé par les moyens dont dispose l'utilisateur (ex. réfrigérateur-congélateur), ce qui entraîne un minimum de contraintes et permet le renouvellement de l'opération une ou plusieurs fois par jour.

Ces deux orthèses mammaires multicellulaires pré-formées et contenant un fluide à haute chaleur latente de congélation peuvent être glissées dans un empiècement porteur, reprenant la forme classique d'un soutien-gorge traditionnel.

L'utilisateur peut employer un soutien-gorge classique (4) de deux tailles supérieures à ses mensurations ou tout autre moyen d'accrochage (bandage, adhésifs, brides élastiques, etc...)

Les meilleurs résultats ont été obtenus avec un empiècement porteur dont la texture de l'enveloppe extérieure, constituée par une couche externe (1), est préférentiellement réalisée en matière synthétique élastique ayant une isothermie supérieure à la couche (2) servant à diffuser le froid par circulation d'air tout en protégeant la peau de gelures éventuelles provoqué par des effets de condensation.

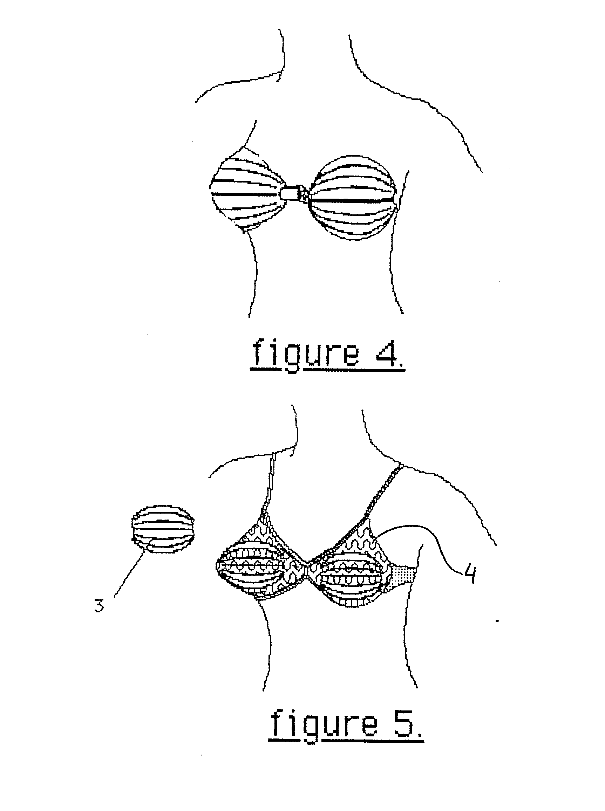

Les orthèses mammaires multi-cellulaires sont formées, dans les différentes versions de l'invention, soit de cellules carrées, rectangulaires, triangulaires ou trapèzoïdales, soit de sphères ou demi-sphères, soit de boudins circulaires superposés et définissant un cône (fig. 4). On peut soit prévoir une forme de bonnet pour chaque type de poitrine, soit définir une forme unique ce qui permet une grande polyvalence dans les applications.

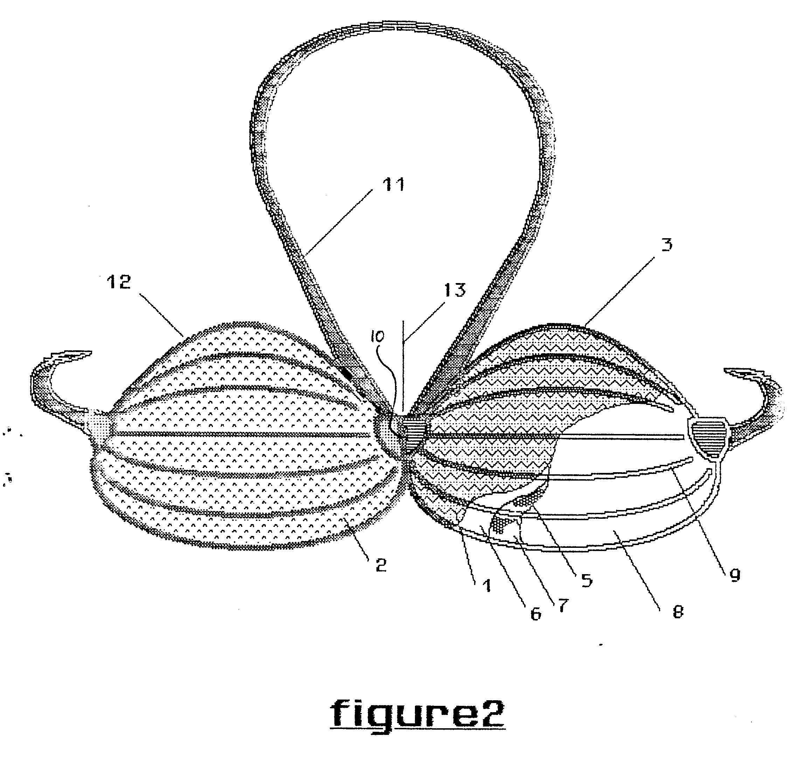

Selon une version préférentielle et conformément à l'invention, les essais nous ont amenés à un dispositif ayant une forme unique en lignes tendues (Fig. 2) les soudures des différents feuillets étant orientées de telle sorte qu'il constituent une structure pré-formée malléable lui permettant d'épouser n'importe quelle forme de type ovoïde, notamment les seins.

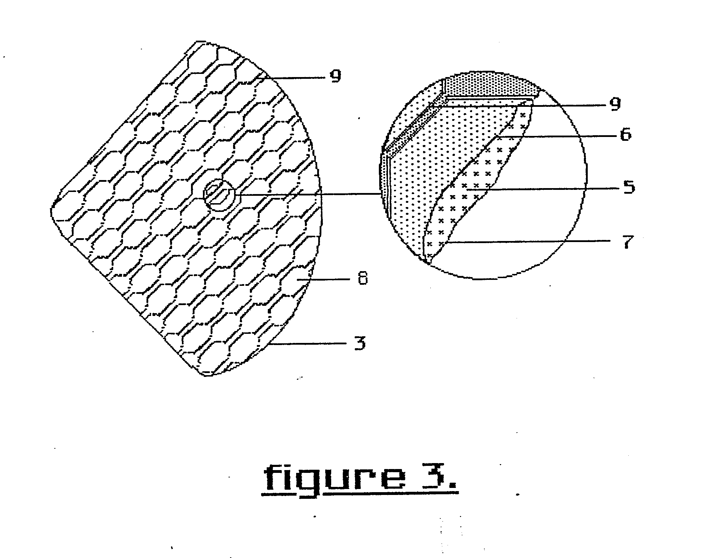

Conformément à l'invention, et comme le montre plus précisément la figure 3, l'orthèse mammaire (3) est formée de cellules contenant une matière congelable (5) ayant une chaleur latente de congélation la plus élevée possible. La figure 3 comporte un grossissement d'une cellule composé de deux feuillets (6-7), soudés partiellement entre eux (9) de façon à créer des cellules (8) permettant de répartir le fluide sur toute la surface de l'orthèse mammaire. Les orthèses mammaires sont réalisées en matière souple résistant au gel. L'orthèse mammaire est pré-formée en usine selon les formes et dimensions habituelles des bonnets de soutien-gorge. Le remplissage de l'orthèse mammaire multicellulaire peut être réalisé à la fabrication en soudant les feuillets (6 et 7) par procédé ultra-sonique, ou réalisé de telle sorte que les cellules communiquent entre elles au niveau de la soudure (9) par un passage permettant un remplissage manuel ou automatique.

Dans une autre version de l'invention, le dispositif est composé d'un empiècement porteur, caractérisé en ce que les feuillets plastiques (6/7) de l'orthèse mammaire multicellulaire et au moins un des textiles synthétiques (1/2) du bonnet (12) sont solidaires par leurs soudures respectives réalisées simultanément à la fabrication par procédé électronique. Reprenant la forme classique d'un soutien-gorge, les bonnets (12) sont remplacés par des orthèses mammaires (3) constituées de cellules (8) contenant un fluide (5) entre deux feuillets (6) et (7) soudés entre eux (9) et constituant un ensemble solidaire de l'empiècement porteur réalisé dans la même matière; l'agrafage (10) situé sur l'axe (13) étant d'un type réglable connu (ex. : bande Velcro, boucle, crochets,...).

L'ensemble utilisé dans ce dispositif est réalisé en polyéthylène ou PVC et le feuillet 7 est préférentiellement en dérivé de polyéthylène et dopé par un agent conducteur connu pour ses propriétés d'échangeur de température entre le fluide contenu dans les cellules de l'orthèse et la peau.

Ainsi réalisé le dispositif de l'invention apporte un confort d'utilisation supérieur et des possibilités de traitements nouveaux par rapport aux systèmes pré-cités. Les différents essais effectués notamment au niveau des seins montre que dès l'application du dispositif, les principes actifs du froid provoquent une vasoconstriction superficielle puis, dans les minutes qui suivent une vasodilatation, en effet l'organisme réagit, pour rétablir la température superficielle, en provoquant un afflux de sang ce qui provoque une augmentation des calories brûlées. Lorsque la température s'élève et que la vasodilatation est à son maximun, la cinétique du réseau macro-moléculaire du feuillet (6) ou (7) de l'orhèse en contact avec la peau se relâche ( propriété connue du PVC soumis à une élévation de température) ; il se produit alors un relargage des principes actifs pouvant être contenus dans le fluide. L'épiderme est à ce moment le plus perméable à un traitement.

Bien entendu, l'invention n'est pas limitée au mode de réalisation représentée sur les dessins annexés. Des modifications restent possibles, notamment du point de vue de la constitution des divers éléments ou par substitution d'équivalents techniques, sans sortir pour autant du domaine de la protection de l'invention. En particulier l'application du dispositif sur tout autre partie du corps chaque fois que qu'un traitement l'exige.

**Revendications**

1 - Dispositif autonome de traitement des seins, du type comportant deux récipients pré-formés amovibles et pouvant contenir un liquide, caractérisé en ce qu'il consiste en au moins une orthèse mammaire (3) dont l'une des faces à un pouvoir isothermique supérieur à l'autre et est constitué par au moins deux feuillets (6-7) en matière plastique souple, déformable, et résistant au gel, soudés partiellement entre eux (9) de façon à créer des cellules (8) dont l'orientation assure une répartition uniforme d'un fluide (5) sur toute la surface de l'orthèse mammaire.

2 - Dispositif selon la revendication 1, caractérisé en ce que l'ensemble est réalisé en polyéthylène et que le feuillet interne (7) est un dérivé de polyéthylène dopé par un agent conducteur connu pour ses propriétés d'échangeur de température.

3- Dispositif selon la revendication 2 caractérisé en ce que l'ensemble est réalisé en d'autres matières susceptibles de répondre aux besoins du traitement.

4- Dispositif selon les revendications 1 à 3 caractérisé en ce l'assemblage, le découpage et le remplissage de l'orthèse mammaire multicellulaire sont réalisés simultanément à la fabrication en soudant les feuillets (6/7) par procédé électronique.

5- Dispositif selon la revendication 1, caractérisé en ce les bonnets (12) préférentiellement réalisés en matière synthétique élastique sont constitués par une couche externe (1) ayant un pouvoir isothermique supérieur à celui de la couche interne (2).

6- Dispositif selon les revendications 1 à 5 caractérisé en ce que les feuillets plastiques (6/7) de l'orthèse mammaire multicellulaire et au moins un des textiles synthétiques (1/2) du bonnet (12) sont solidaires par leurs soudures respectives réalisées simultanément à la fabrication par procédé électronique.

7- Dispositif selon les revendications de 1 à 6 caractérisé en ce que les soudures des différents feuillets sont orientés de telle sorte qu'ils constituent une structure pré-formée malléable lui permettant d'épouser n'importe quelle forme de type ovoïde, notamment les seins.

8 - Dispositif selon la revendication 1, caractérisé en ce que le fluide (5) est à haute chaleur latente de congélation rechargeable.

9 - Application du dispositif selon l'une quelconque des revendications précédentes, à un traitement raffermisseur et/ou vasoconstricteur et/ou décongestionnant par migration des principes actifs au travers de l'orthèse.

0263041

**figure 1.**

0263041

**figure2**

0263041

## figure 3.

0263041

figure 4.

figure 5.